# EUROPEAN PATENT SPECIFICATION

(11) **EP 0 475 182 B1**
(45) Date of publication and mention of the grant of the patent: **05.12.2001**
(21) Application number: 91114296.6
(22) Date of filing: 26.08.1991
(51) Int. Cl.: C12N 15/51, C12N 15/62

(54) **Method for protein synthesis using CKS fusion proteins**
Verfahren zur Herstellung von Proteinen durch Verwendung von CKS-Fusionsproteinen
Procédé pour la production de protéines en utilisant des protéines fusionnées de type CKS

(30) Priority: 24.08.1990 US 573103
(43) Date of publication of application: 18.03.1992
(73) Proprietor: ABBOTT LABORATORIES, Abbott Park, Illinois 60064-3500 (US)
(72) Inventor: Bolling, Timothy Jon, Gurnee, Illinois 60031 (US); Mandecki, Wlodzimierz, Libertyville, Illinois 60048 (US)
(74) Representative: Modiano, Guido, Dr.-Ing.

(56) References cited:
- EP-A- 0 331 961
- EP-A- 0 461 462
- EP-A- 0 472 207
- WO-A-89/04669

## Description

This invention refers to a CKS method of protein synthesis.

CKS methods of protein synthesis are known in the art, for inst. a CKS method has been disclosed in EP-A- 0331 961.-

With this known method certain proteins were expressed. It did not occur to the man skilled in the art, that such method could be particularly convenient in expressing HCV proteins.

It has now been found that particularly good results are achieved in providing a method for expressing HCV protein in a microbe, which method is characterized by the steps of :
(a) providing a DNA vector having a control region, a region encoding at least a portion of CKS protein, and a region encoding said HCV protein, said control region directing expression of said coding regions;
(b) transforming said microbe with said DNA vector; and
(c) expressing a fusion protein of at least a portion of CKS and said HCV protein.-

It has also been found that a particularly advantageous cloning vector for transforming cells to express heterologous HCV protein can be obtained, when such vector is characterized by a plasmid having a control region, a region encoding at least a portion of CKS, and a region encoding for said HCV protein.

Within the frame of the invention, said HCV protein is characterized in that it is produced by:
(a) providing a DNA vector having a control region, a region coding at least a portion of CKS enzyme, and a region coding for HCV protein;
(b) transforming a cell to which said HCV protein is heterologous with said DNA vector;
(c) cloning said cell;
(d) fermenting said cloned cells;
(e) recovering a fusion product of CKS and said HCV protein; and
(f) cleaving the CKS from said HCV protein in the fusion product.

According to another aspect of the invention a gene sequence for insertion into a plasmid vector is provided for, which gene sequence is characterized by:
(a) a promoter;
(b) a ribosome binding site;
(c) a first gene subsequence encoding at least a portion of CKB; and
(d) a second gene subsequence encoding a HCV protein sequence.
The invention will be more apparent from the description of two examples embodying auch invention, which examples in turn refer to the enclosed drawings, in which
Fig. 1 diagrammatically shows the construction of Plasmid pHCV-34.
Fig. 2 shows the complete DNA sequence of pHCV-34, in which the predicted amino acid sequence of the structural gene is included with the DNA sequence.
Fig. 3 shows the recombinant protein encoded by pHCV-34.-
Fig. 4 presents the expression of pHCV-34 proteins in E.coli.-
Fig. 5, 6 and 7 diagrammatically show the construction of plasmid pHCV-23, of plasmid pHCV-29 and of plasmid pHCV- 31 respectively.-
Fig. 8 shows the complete DNA sequence of pHCV-31, the predicted amino acid sequence of the structural gene being included with the DNA sequence.-
Fig. 9 shows the recombinant protein encoded by pHCV-31.
Fig. 10, 11, and 12 presents the expression of the recombinant fusion proteins CKS-33c, CKS-BCD and CKS-33-BCD respectively.-
In now describing the examples, some definitions of plasmids, promoters, vectors, and in general CKS method steps will be used, which are more particularly described in EP-A- 0 331 961, which enjoys common ownership, and accordingly such definitions are herewith incorporated by reference.

### EXAMPLE 1- CKS-CORE

### A. Construction of the Plasmid pJ0200. -

The cloning vactor pJ0200 allows the fusion of recombinant proteins to the CKS protein. The plasmid consists of the plasmid pBR322 with a modified lac promoter fused to a KdsB gene fragment (encoding the first 239 of the entire 248 amino acids of the E.coli CMP-KDO synthetase of CKS protein), and a synthetic linker fused to the end of the KdsB gene fragment. The cloning vector pJ0200 is a modification of vector pTB210.- The synthetic linker includes: multiple restriction sites for insertion of genes; translational stop signals, and the trpA rho-independent transcriptional terminator.-

### 8. Preparation of HCV CKS-Core Expression Vector

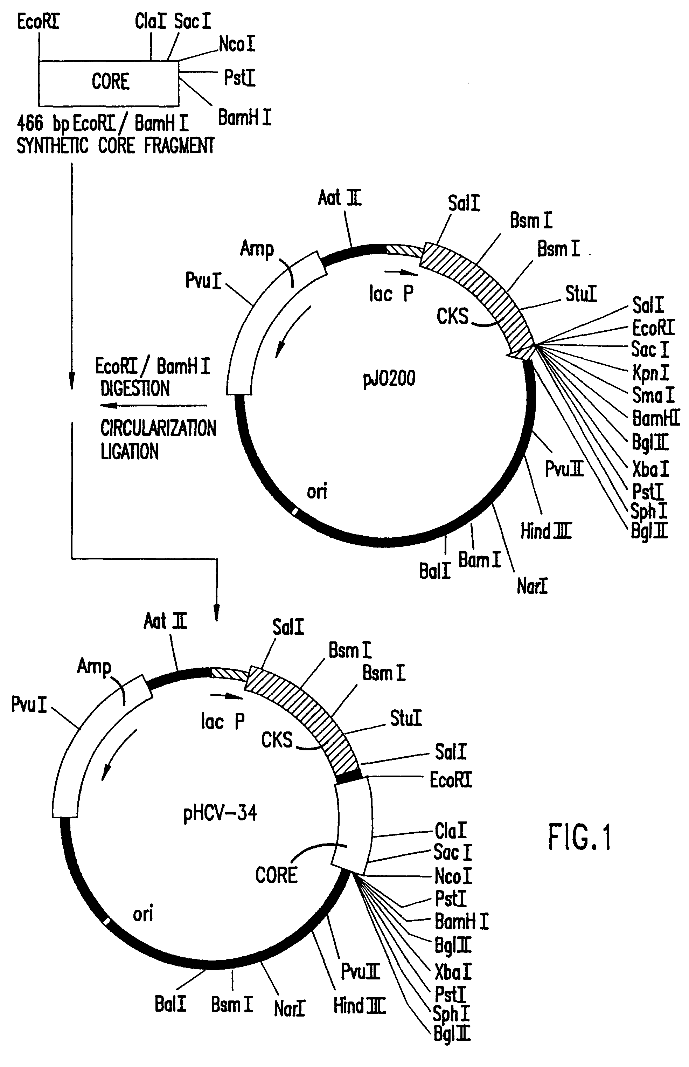

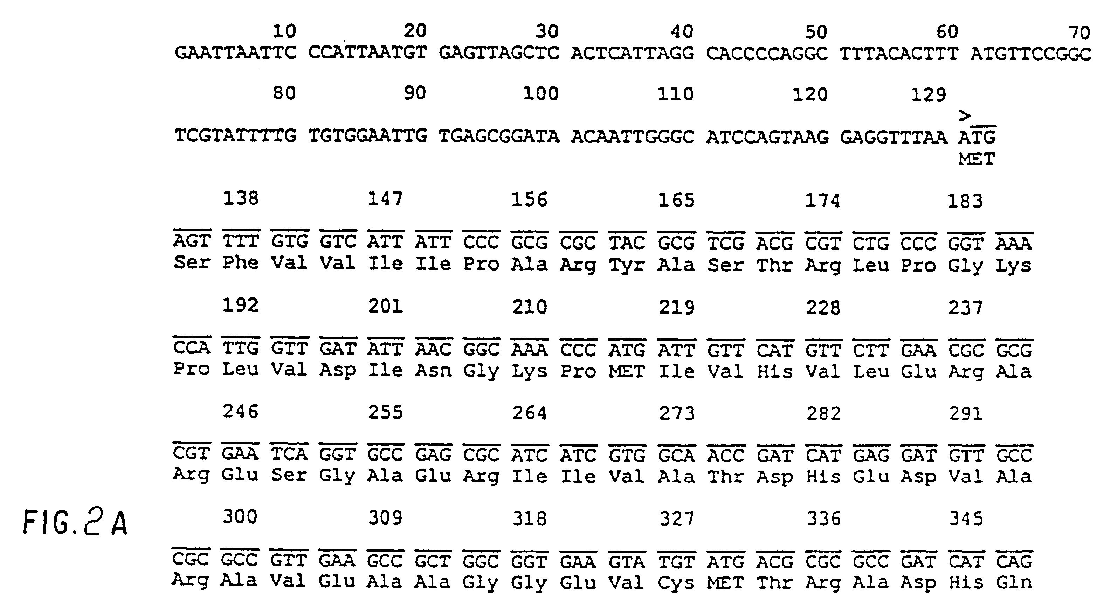

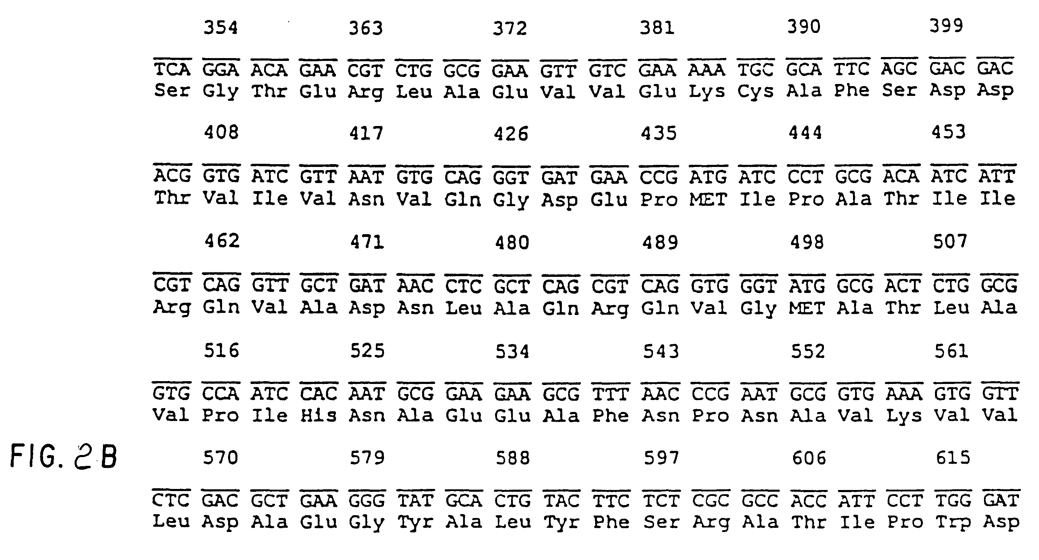

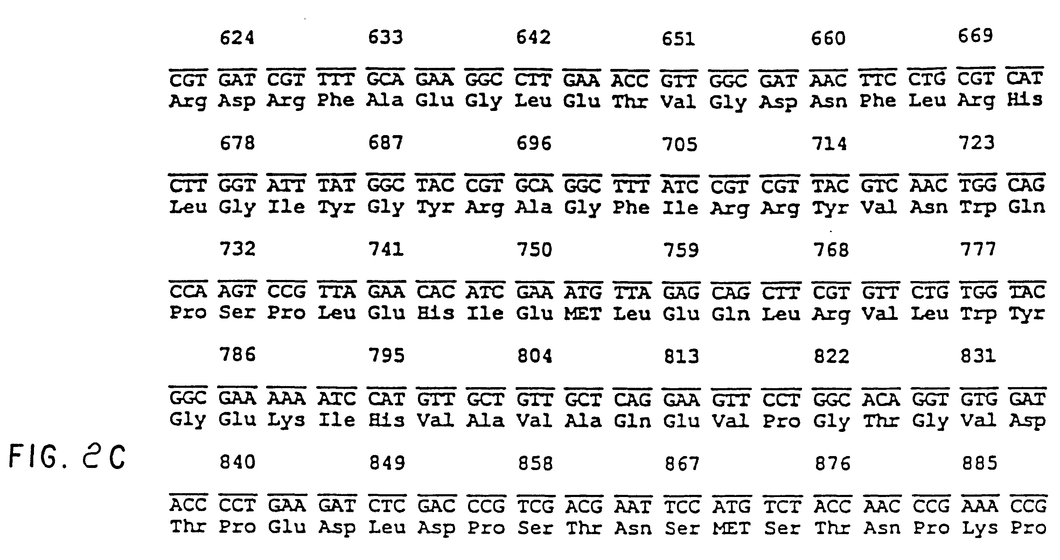

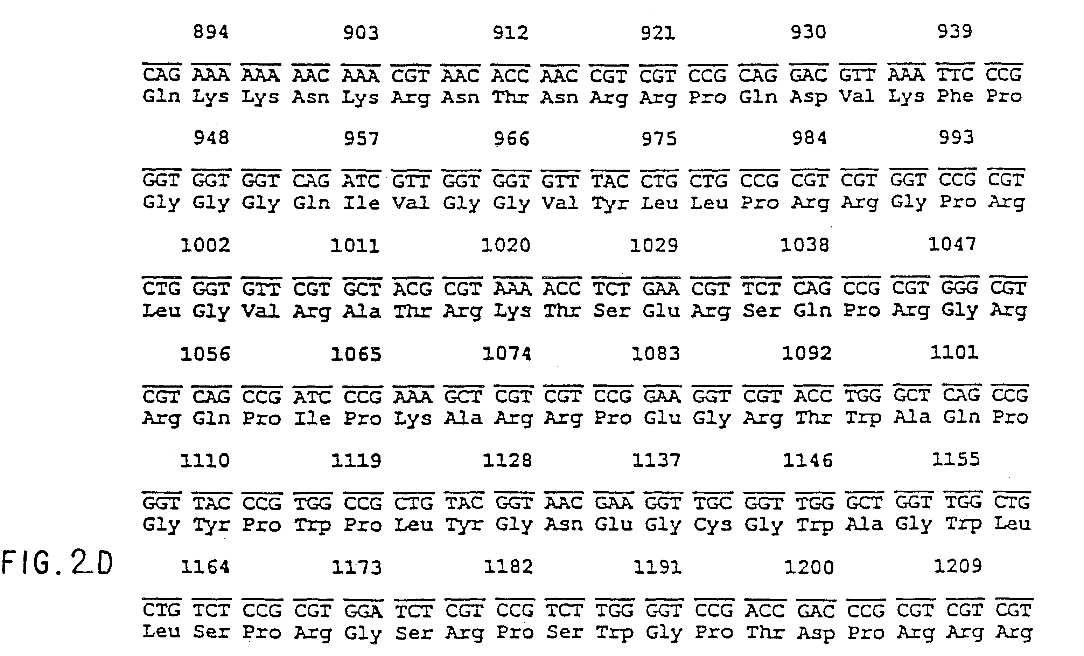

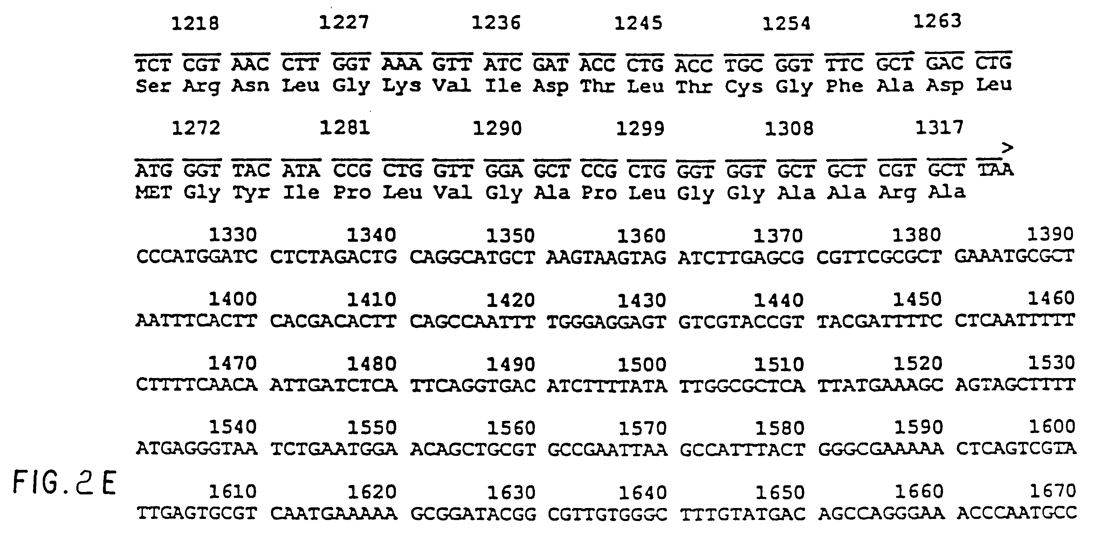

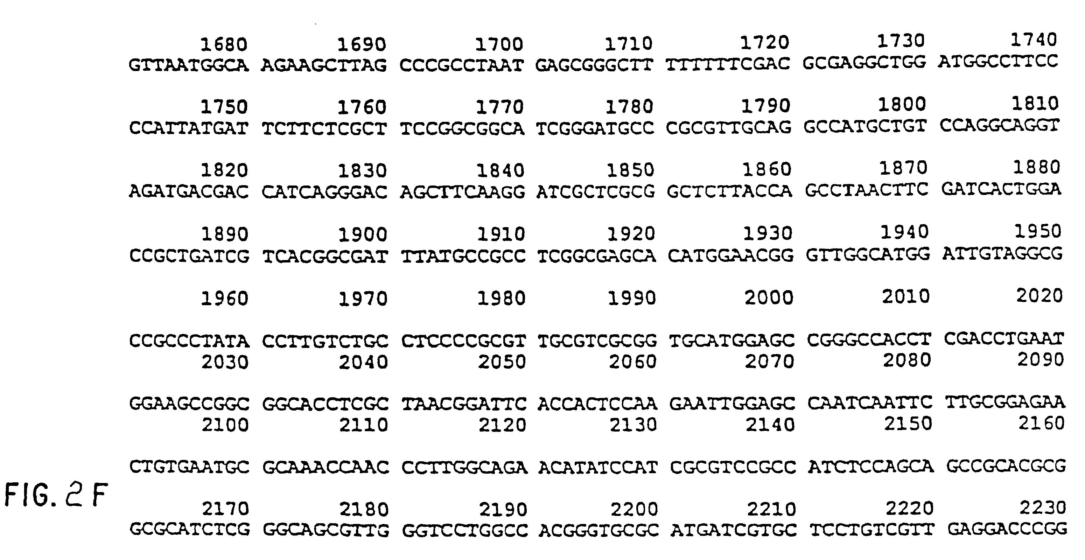

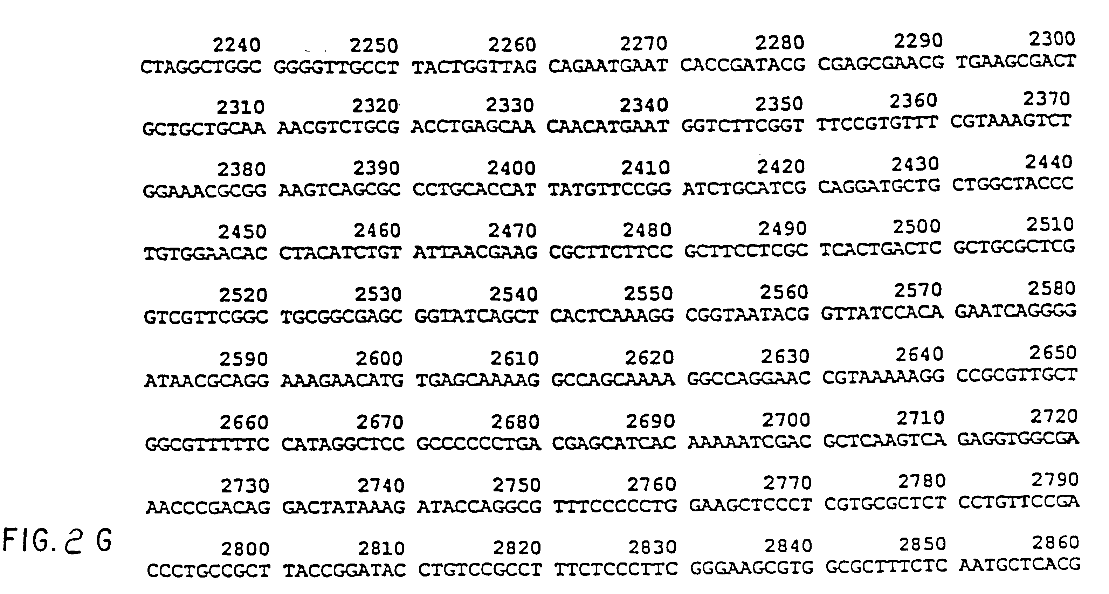

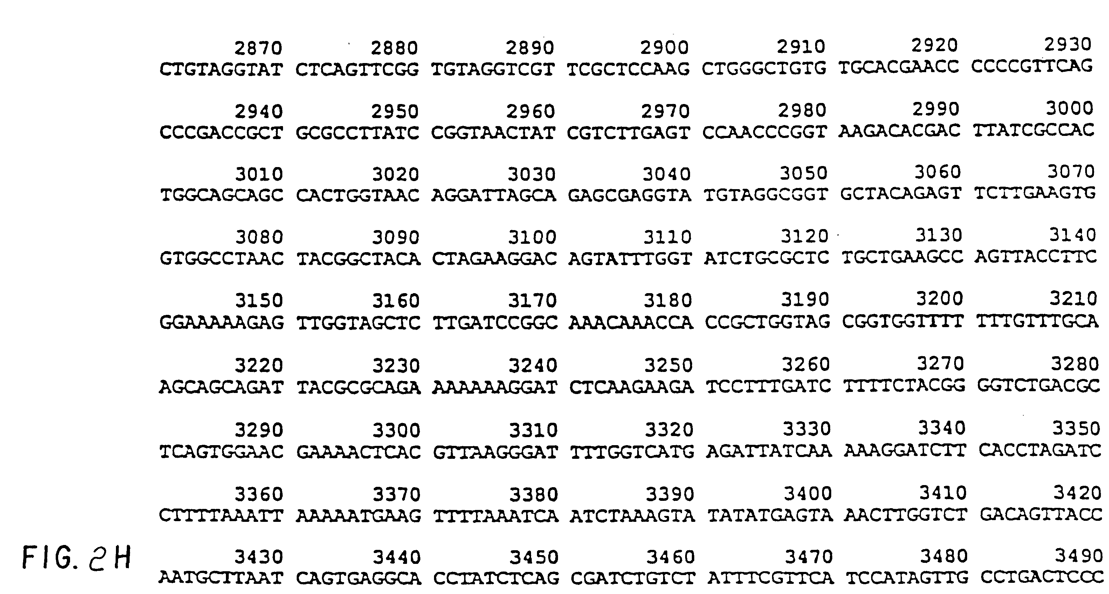

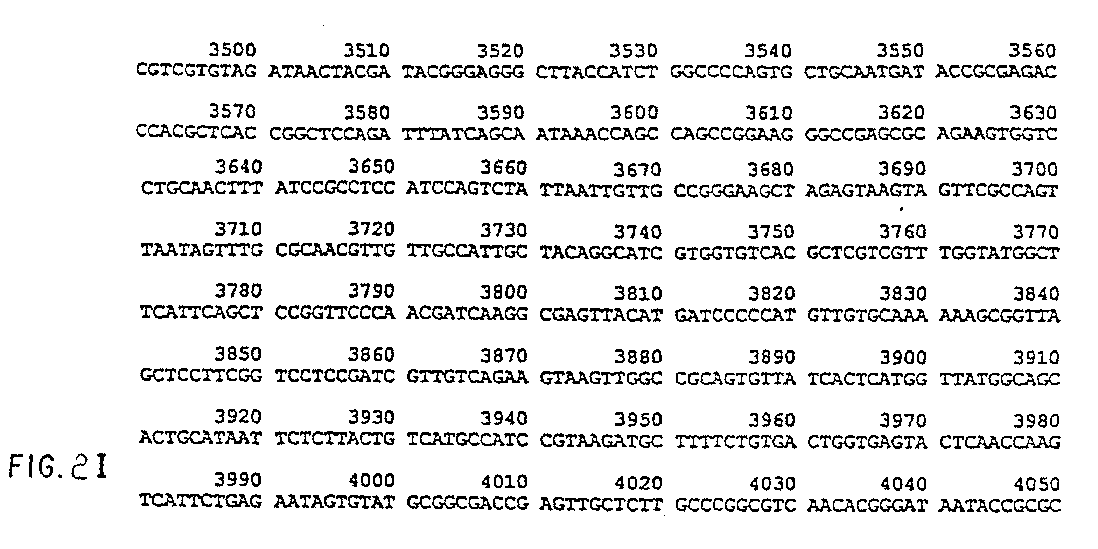

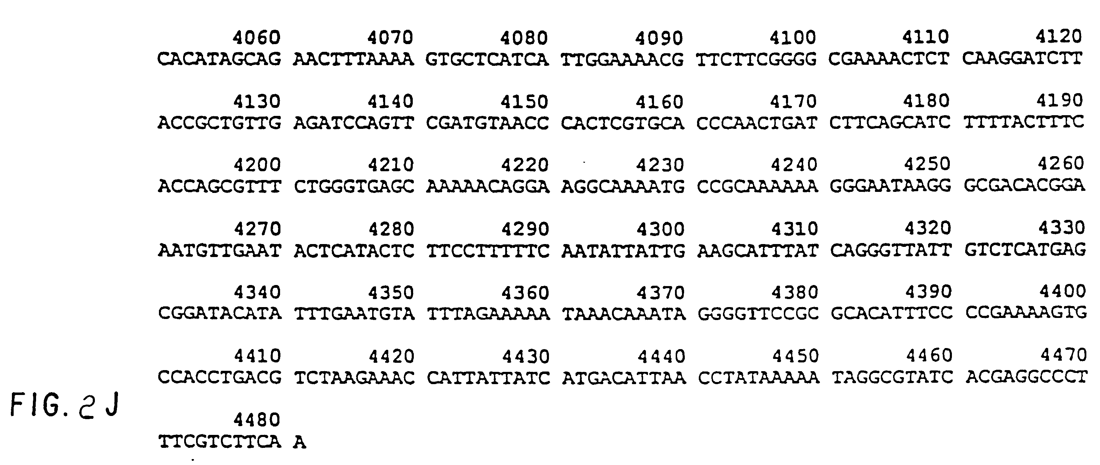

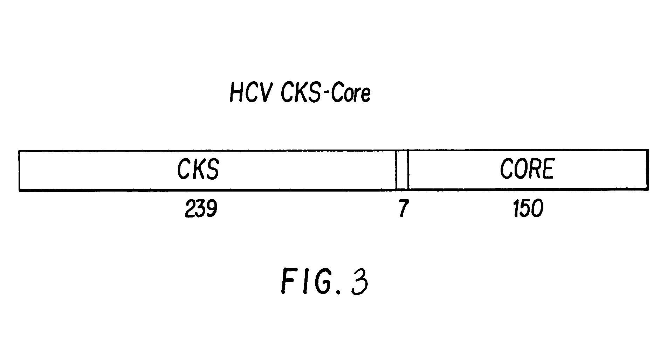

Six individual nucleotides representing amino acids 1-150 of the HCV genome were ligated together and cloned as a 466 base pair EcoR1-BamH1 fragment into the CKS fusion vector pJ0200 as presented in Figure 1. The complete DNA sequence of this plasmid, designated pHCV-34, and the entire amino acid sequence of the pHCV-34 recombinant antigen produced is presented in Figure 2 . The resultant fusion protein HCV CKS-Core, consists of 239 amino acids of CKS, seven amino acids contributed by linker DNA sequences, and the first 150 amino acids of HCY as illustrated in Figure 3.

The pHCV-34 plasmid and the CKS plasmid pTB210 were transformed into E. coli K-12 strain xL-1 (recA1, endA1, gyrA96, thi-1, hsdR17, supE44, relA1, lac/F', proAB, laclqZDM15, TN10) cells made competent by the calcium choride method. In these constructions the expression of the CKS fusion proteins was under the control of the lac promoter and was induced by the addition of IPTG. These plasmids replicated as independent elements, were nonmobilizable and were maintained at approximately 10-30 copies per cell.

### C. Characterization of Recombinant HCV-Core

In order to establish that clone pHCV-34 expressed the unique HCV-CKS Core protein, the pHCV-34/XL-1 culture was grown overnight at 37 C in growth media consisting of yeast extract, trytone, phosphate salts, glucose, and ampicillin. When the culture reached an OD600 of 1.0, IPTG was added to a final concentration of 1mM to induce expression. Samples (1.5 ml) were removed at 1 hour intervals, and cells were pelleted and resuspended to an 00600 of 1.0 in 2X SDS/PAGE loading buffer. Aliquots (15ul) of the prepared samples were separated on duplicate 12.5% SDS/PAGE gels.

One gel was fixed in a solution of 50% methanol and 10% acetic acid for 20 minutes at room temperature, and then stained with 0.25% Coomassie blue dye in a solution of 50% methanol and 10% acetic acid for 30 minutes. Destaining was carried out using a solution of 10% methanol and 7% acetic acid for 3-4 hours, or until a clear background was obtained.

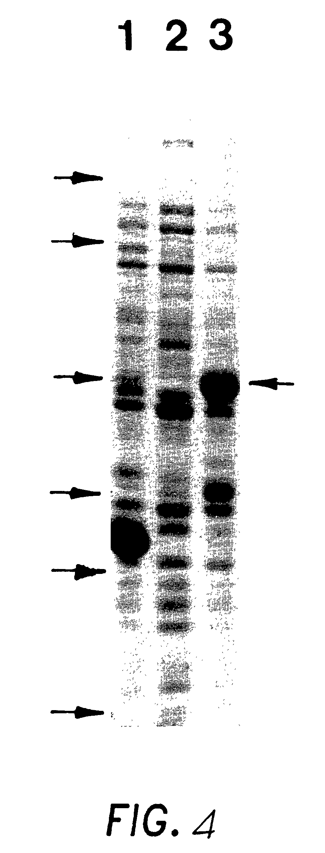

Figure 4 presents the expression of pHCV-34 proteins in E. coli. Molecular weight standards were run in Lane M. Lane 1 contains the plasmid pJ0200-the CKS vector without the HCV sequence. The arrows on the left indicate the mobilities of the molecular weight markers from top to bottom : 110,000; 84,000; 47,000; 33.000; 24,000; and 16,000 daltons. The arrows on the right indicate the mobilities of the recombinant HCV proteins. Lane 2 contains the E. coli lysate containing pHCV-34 expressing CKS-Core (amino acids 1 to 150) prior to induction; and Lane 3 after 3 hours of induction. The results show that the recombinant protein pHCV-34 has an apparent mobility corresponding to a molecular size of 48,000 daltons. This compares acceptably with the predicted molecular mass of 43,750 daltons.

Proteins from the second 12.5% SDS/PAGE gel were electrophoretically transferred to nitrocellulose for immunoblotting. The nitrocellulose sheet containing the transferred proteins was incubated with Blocking Solution for one hour and incubated overnight at 4 C with HCV patients' sera diluted in TBS containing E.coli K-12 strain XL-1 lysate. The nitrocellulose sheet was washed three times in TBS, then incubated with HRPO-labeled goat anti-human IgG, diluted in TBS containing 10% fetal calf sera. The nitrocellulose was washed three times with TBS and the color was developed in TBS containing 2 mg/ml 4-chloro-1napthol, 0.02% hydrogen peroxide and 17% methanol. Clone HCV-34 demonstrated a strong immunoreactive band at 48,000 daltons with the HCV patients' sera. Thus, the major protein in the Coomassie stained protein gel was immunoreactive, Normal human serum did not react with any component of pHCV-34.

### EXAMPLE 2 HCV CKS-33C-BCD

### A. Preparation of HCV CKS-33c-BCD Expression Vector

The construction of this recombinant clone expressing the HCV CKS-33-BCD antigen was carried out in three steps described below. First, a clone expressing the HCV CKS-BCD antigen was constructed, designated pHCV-23. Second, a clone expressing the HCV CKS-33 antigen was constructed, designated pHCV-29. Lastly, the HCV BCD region was excised from pHCV-23 and inserted into pHCV-29 to construct a clone expressing the HCV CKS-33-BCD antigen, designated pHCV-31.

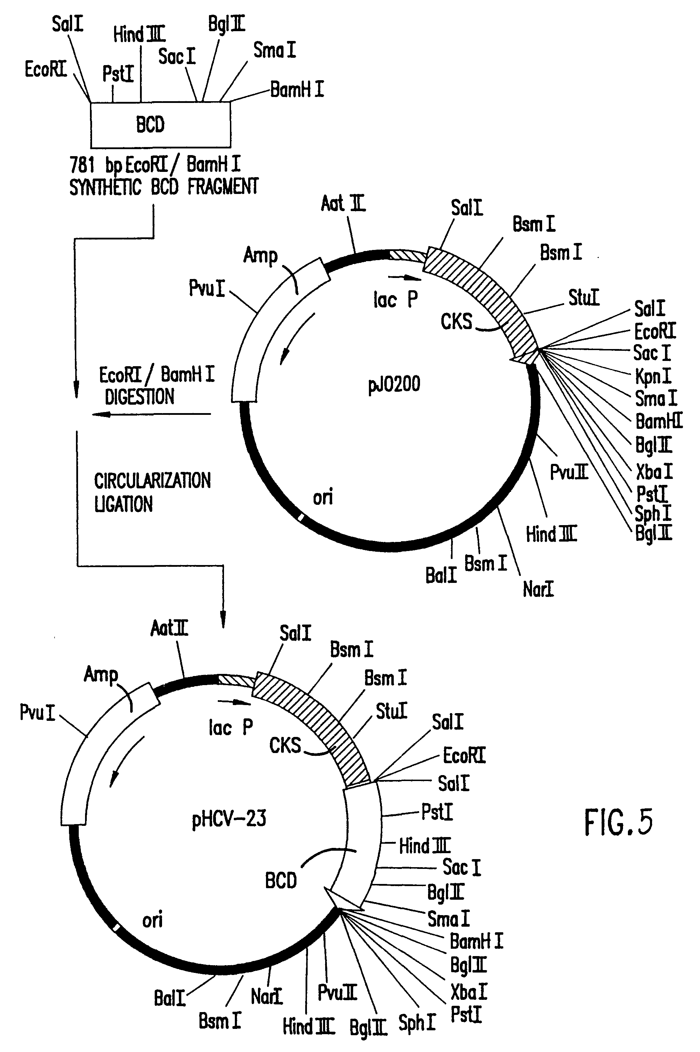

To construct the plasmid pHCV-23, thirteen individual oligonucleotides representing amino acids 1676-1931 of the HCV genome were ligated together and cloned as three separate EcoR1-BamH1 subfragments into the CKS fusion vector pJ0200. After subsequent DNA sequence confirmation, the three subfragments, designated B, C, and 0 respectively, were digested with the appropriate restriction enzymes, gel purified, ligated together, and cloned as a 781 base pair EcoR1-BamH1 fragment in the CKS fusion vector pJ0200, as illustrated in Figure 5. The resulting plasmid, designated pHCV-23, expresses the HCV CKS-BCD antigen under control of the lac promoter. The HCV CKS-BCD antigen consists of 239 amino acids of CKS, seven amino acids contributed by linker DNA sequences, 256 amino acids from the HCV NS4 region (amino acids 1676-1931, and 10 additional amino acids contributed by linker DNA sequences).

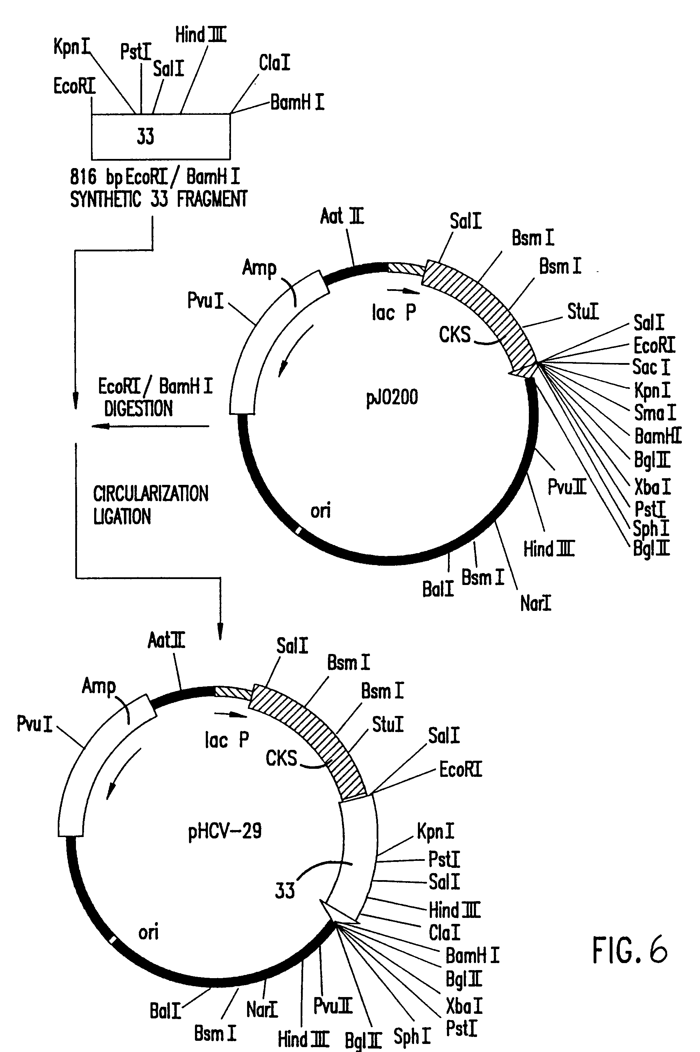

To construct the plasmid pHCV-29 twelve individuel oligonucleotides representing amino acids 1192-1457 of the HCV genome were ligated together and cloned as two separate EcoR1-BamH1 subfragments in the CKS fusion vector pJ0200. After subsequent DNA sequence confirmation, the two subfragments were digested with the appropriate restriction enzymes, gel purified, ligated together and cloned again as an 816 base pair EcoR1-BamH1 fragment in the CKS fusion vector pJ0200, as illustrated in Figure 6. The resulting plasmid, designated pHCV-29, expresses the CKS-33 antigen under control of the lac promoter. The HCV CKS-33 antigen consists of 239 amino acids of CKS, eight amino acids contributed by linker ONA sequences, and 266 amino acids form the HCV NS3 region (amino acids 1192-1457).

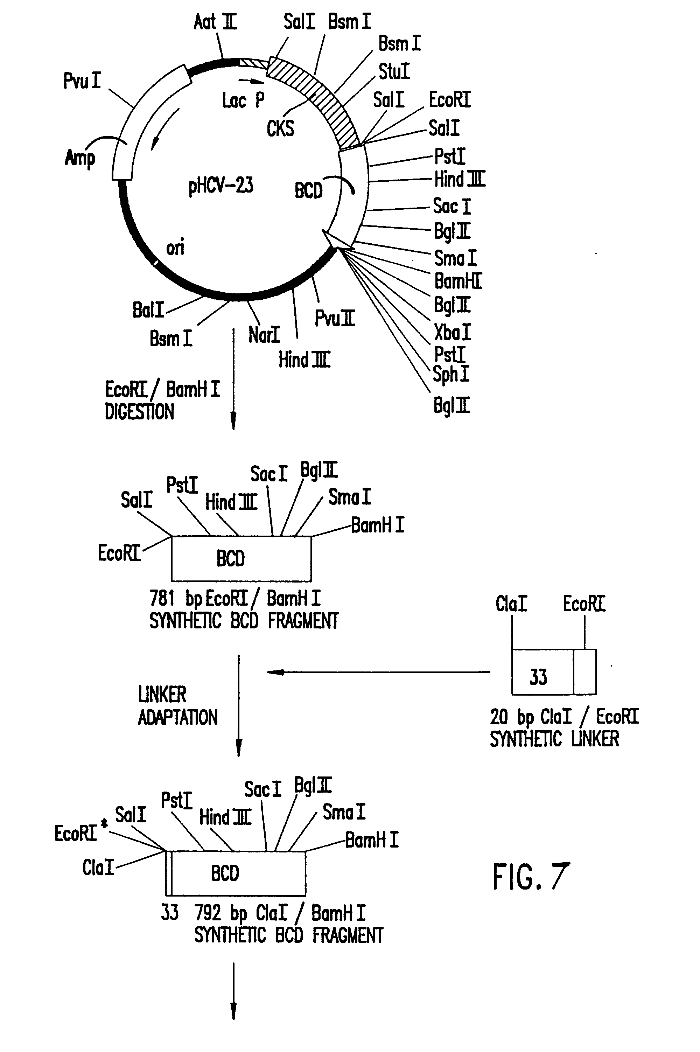

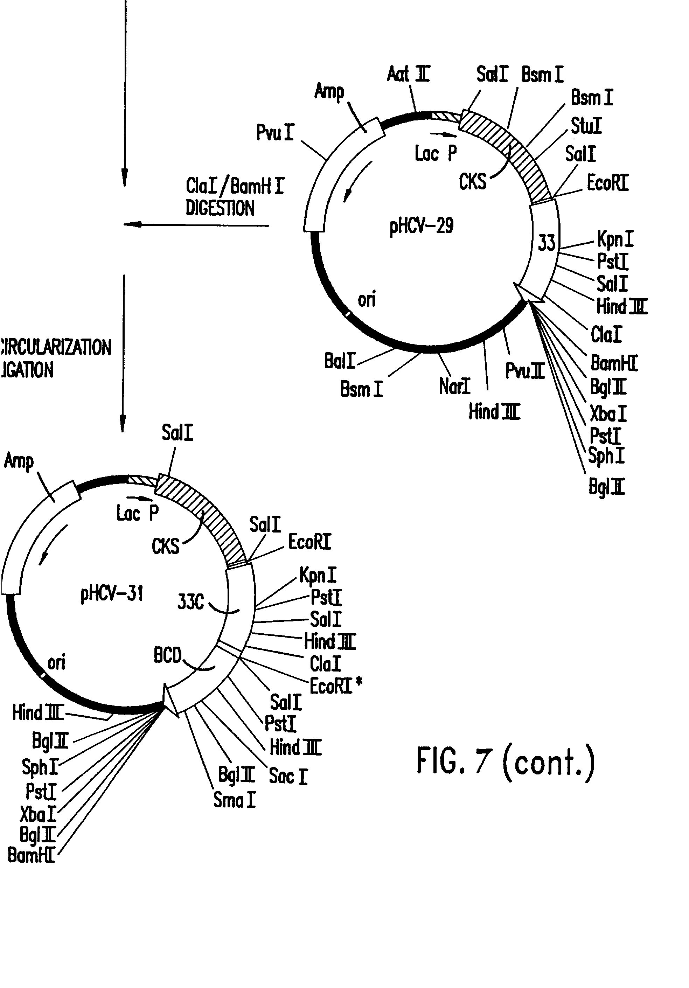

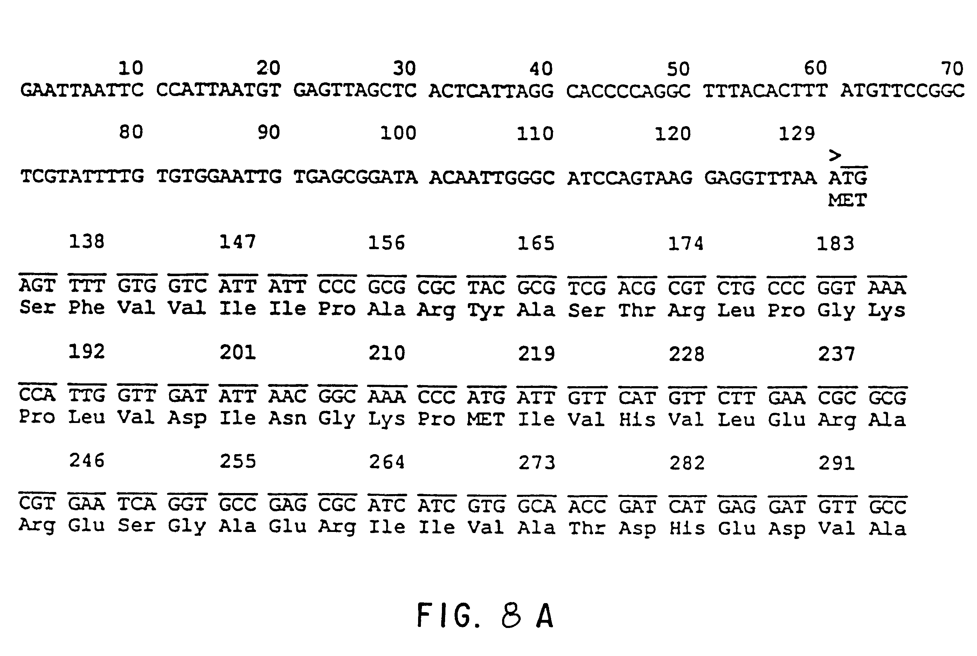

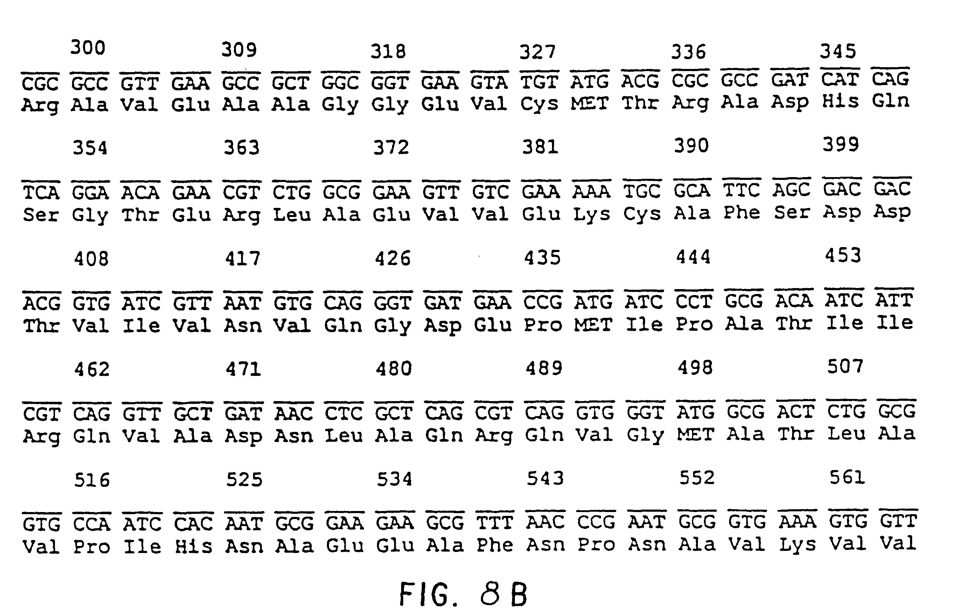

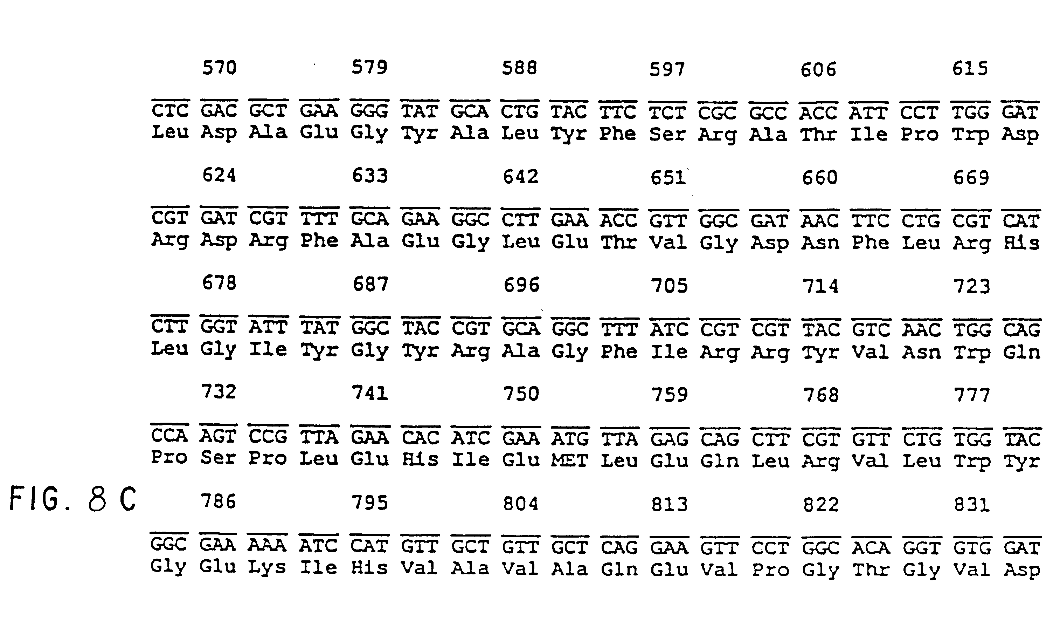

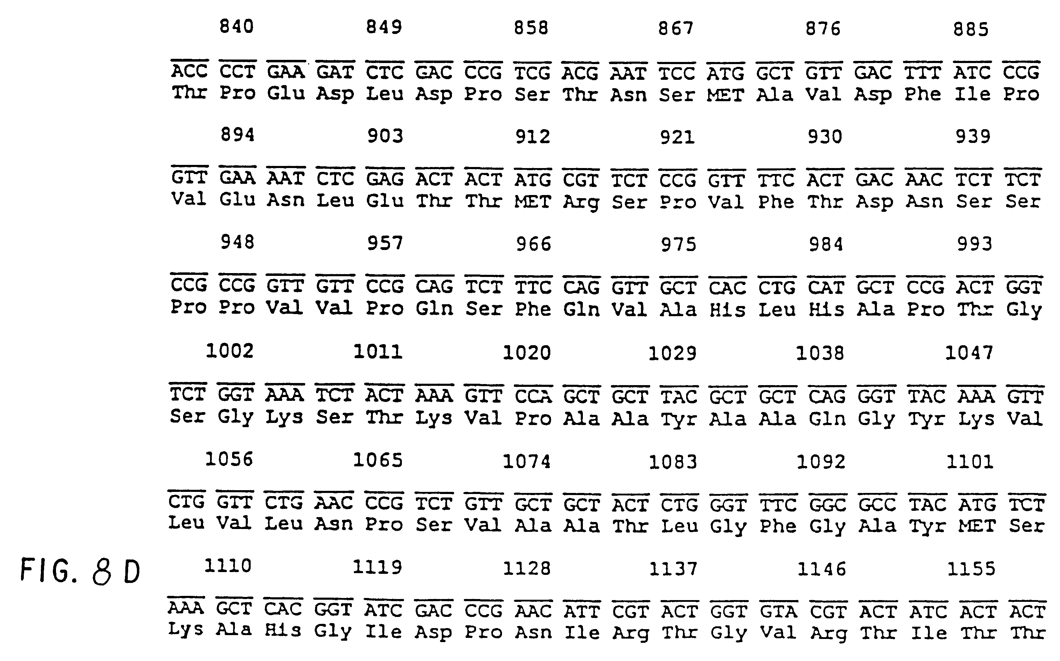

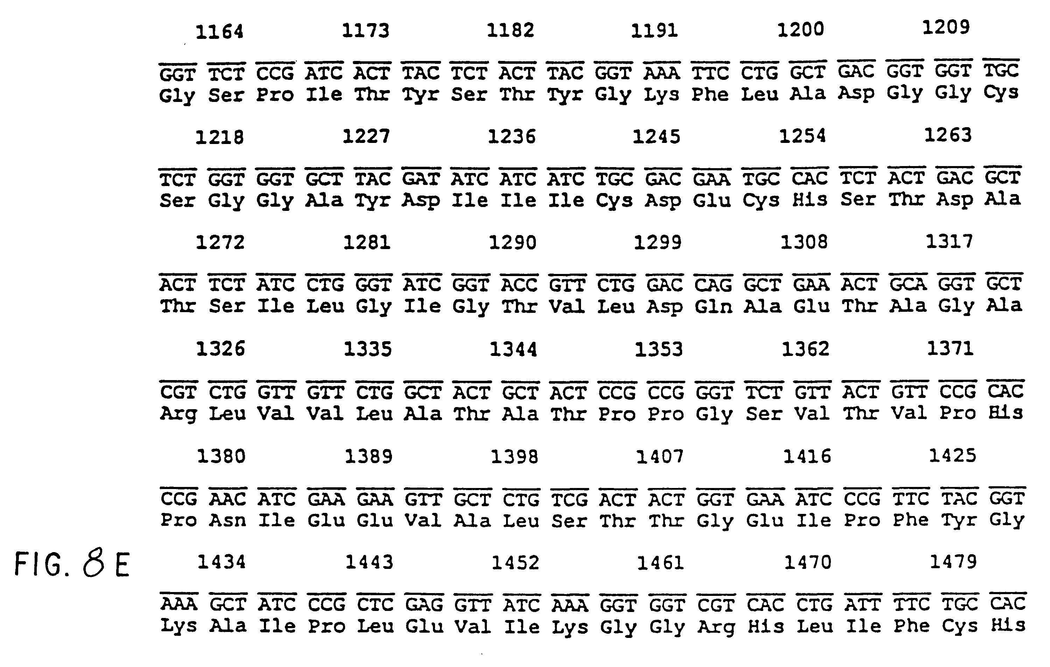

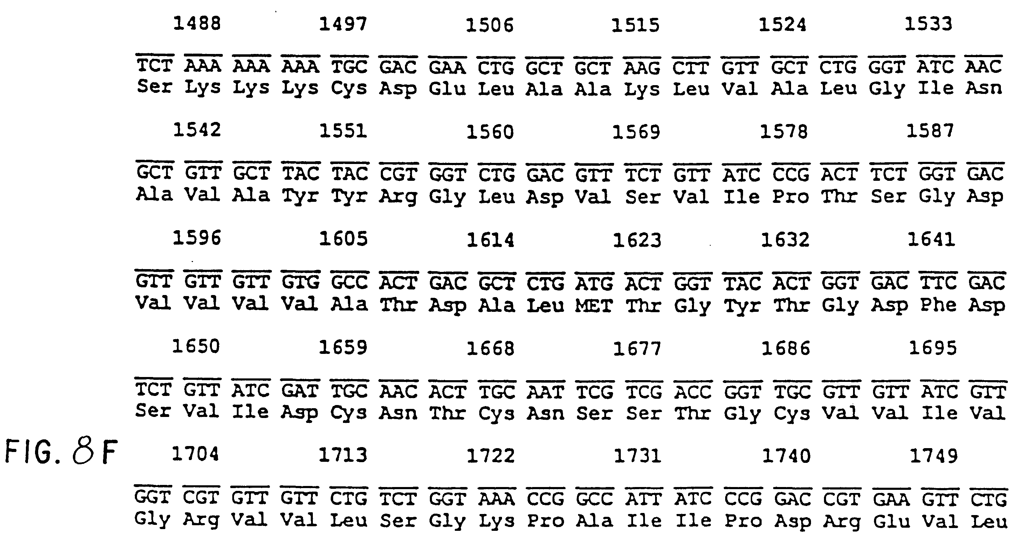

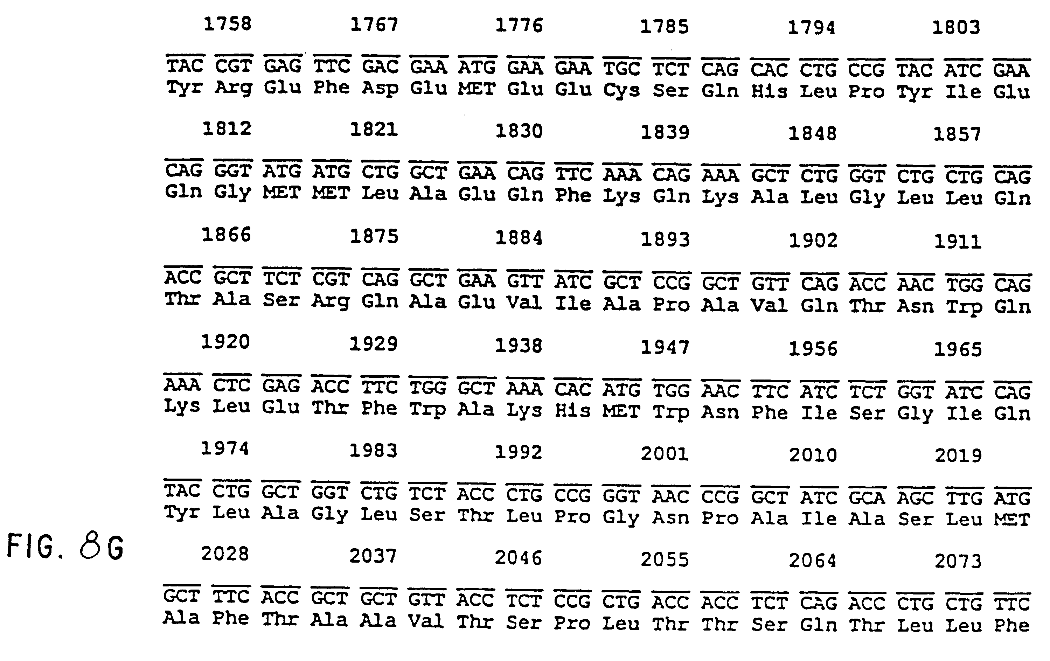

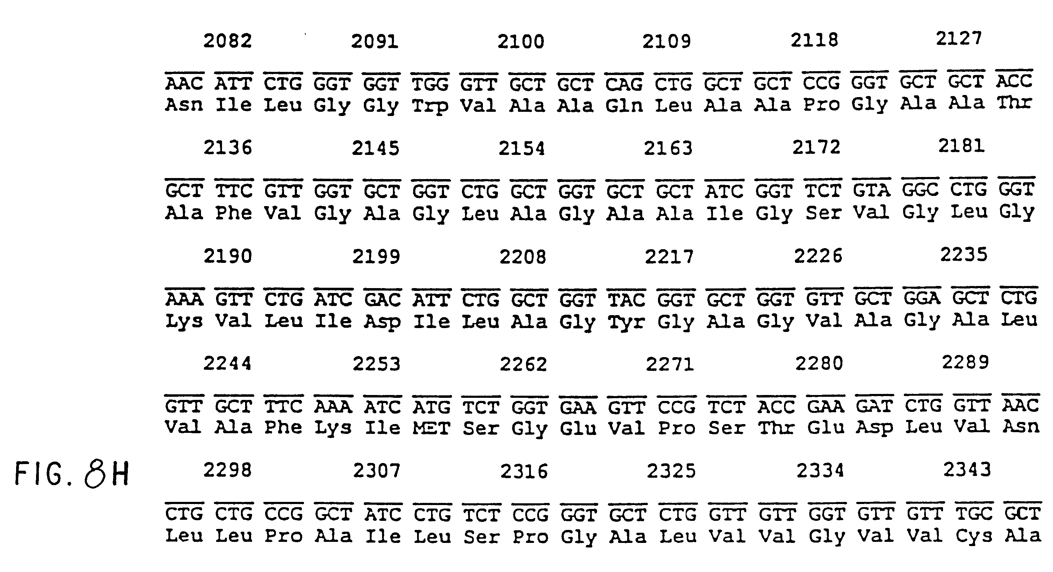

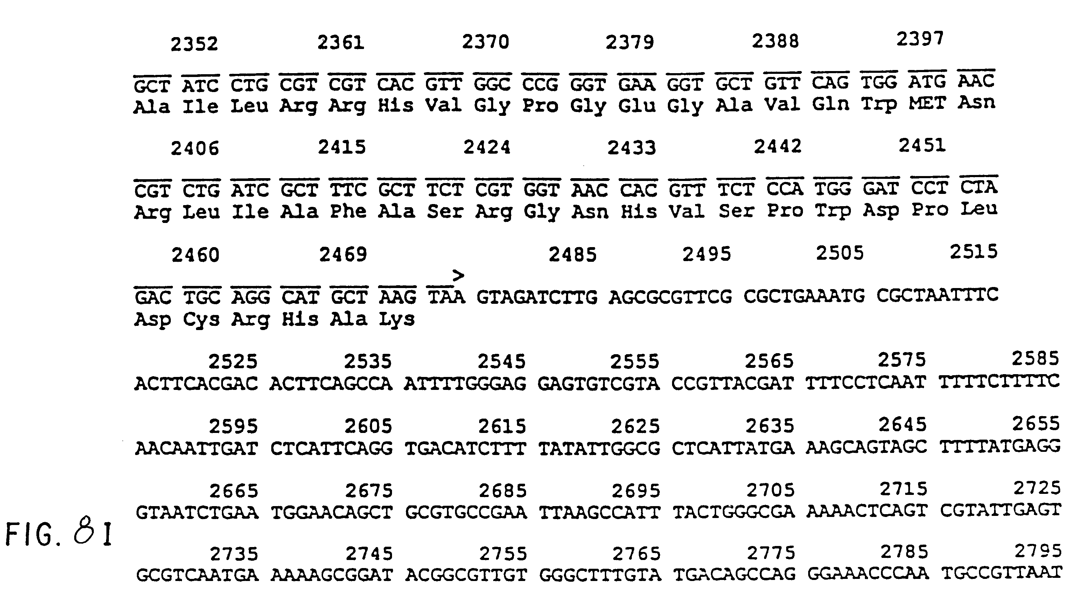

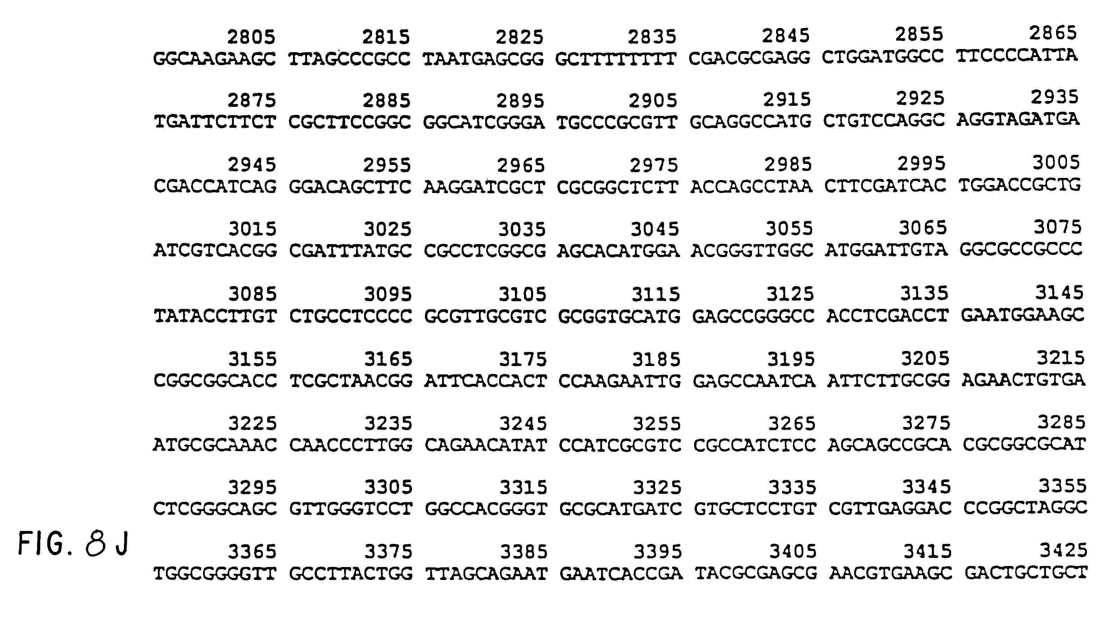

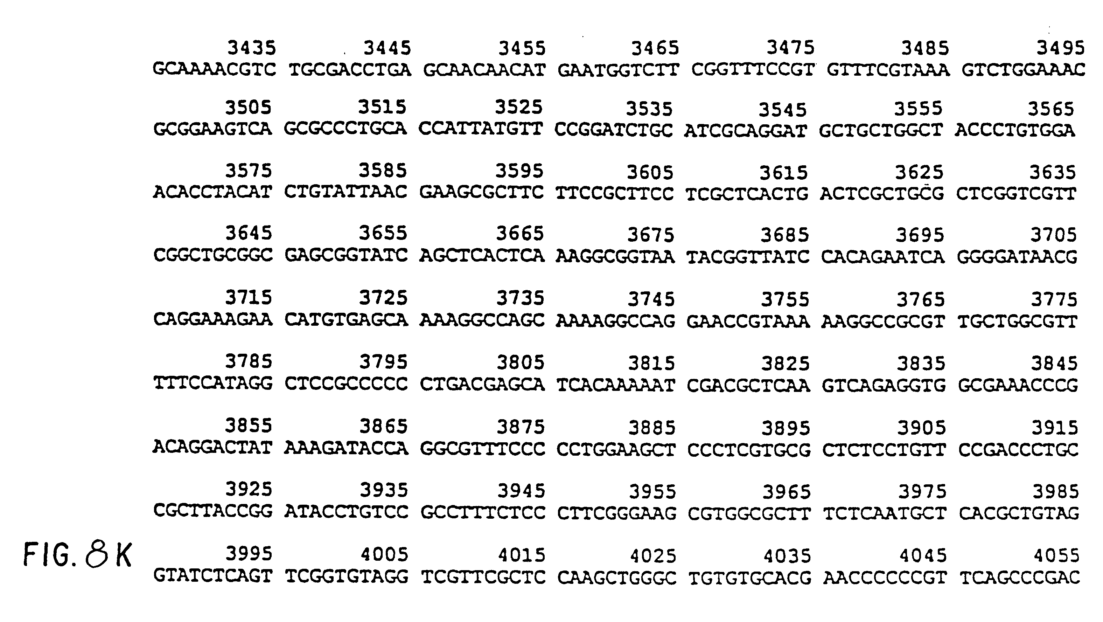

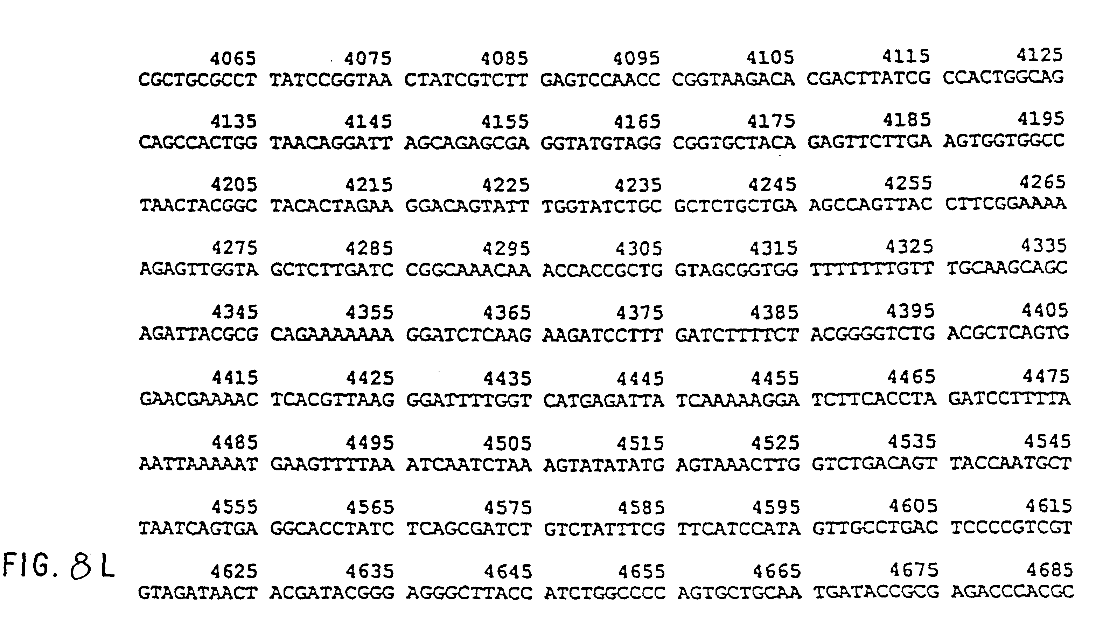

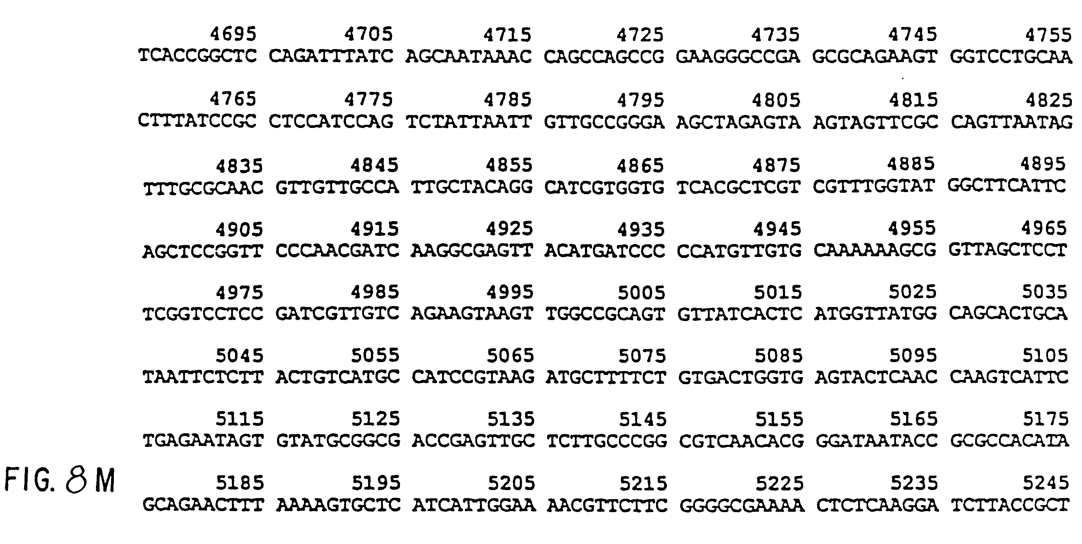

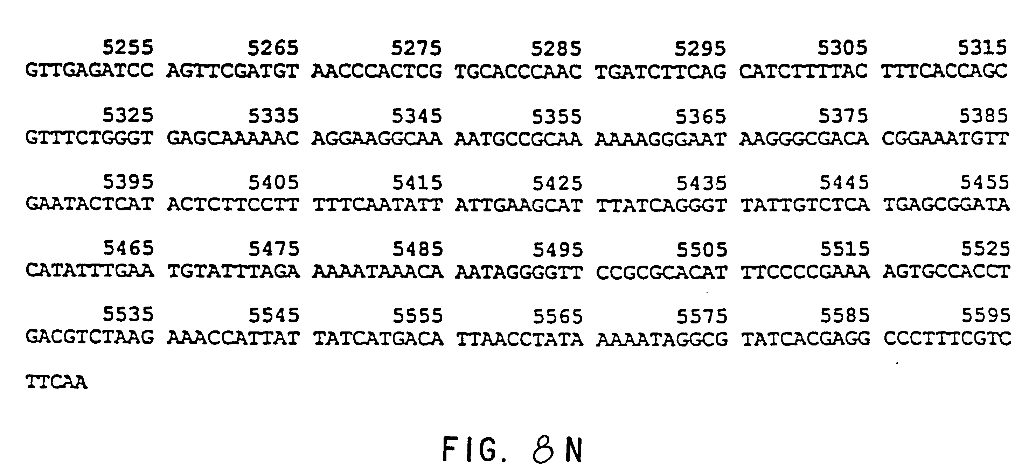

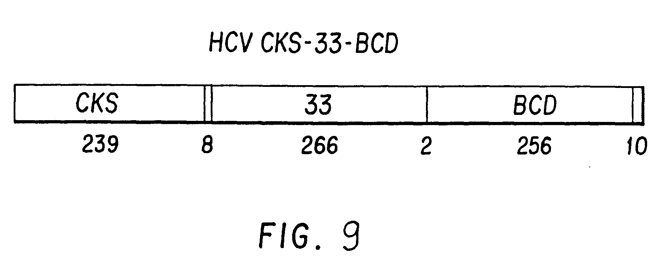

To construct the plasmid pHCV-31, the 781 base pair EcoR1-BamH1 fragment from pHCV-23 representing the HCV-BCD region was linker-adapted to produce a Cla1-BamH1 fragment which was then gel purified and ligated into pHCV-29 at the Cla1-BamH1 sites as illustrated in Figure 7 . The resulting plasmid, designated pHCV-31, expresses the pHCV-31 antigen under control of the lac promoter. The complete DNA sequence of pHCV-31 and the entire amino acid sequence of the HCV CKS-33-BCD recombinant antigen produced is presented in Figure 8 . The HCV CKS-33-8CD antigen consists of 239 amino acids of CKS, eight amino acids contributed by linker DNA sequences, 266 amino acids of the HCV NS3 region (amino acids 1192-1457), 2 amino acids contributed by linker DNA sequences, 256 amino acids of the HCV NS4 region (amino acids 1676-1931), and 10 additional amino acids contributed by linker DNA sequences. Figure 9 presents a schematic representation of the pHCV-31 antigen.

The pHCV-31 plasmid was transformed into E.coli K-12 strain XL-1 in a manner similar to the pHCV-34 and CKS-pTB210 plasmids of Example 1 of EP - A - 0331 961.-

### 8 Characterization of Recombinant HCV CKS-33-BCD

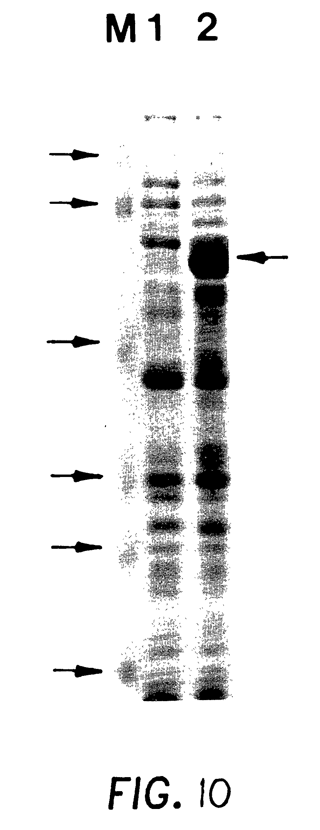

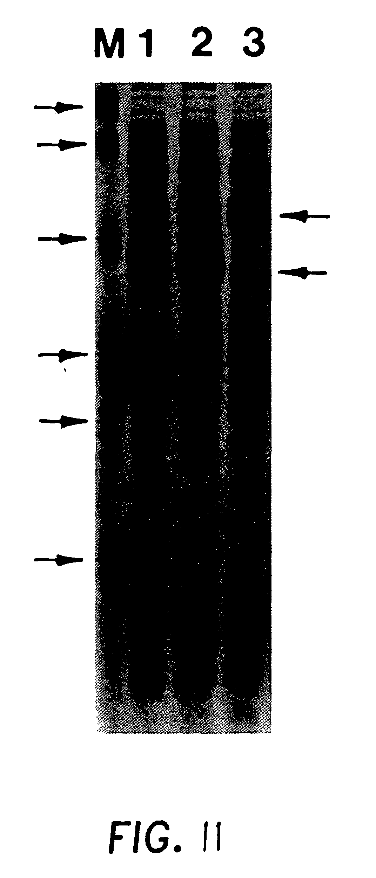

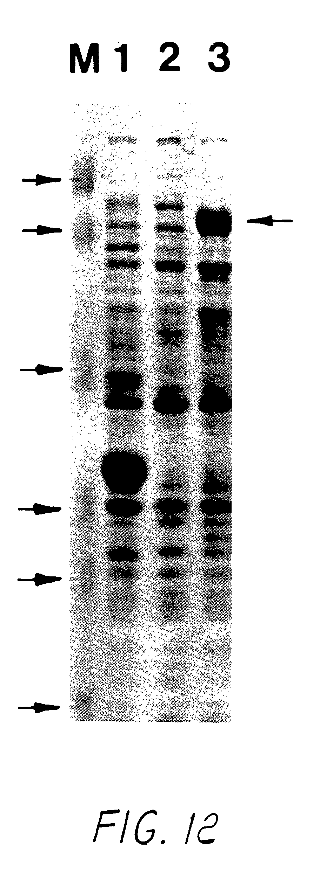

Characterization of pHCV CKS-33-SCD was carried out in a manner similar to pHCV CKS-Core of Example 1. pHCV-23, pHCV SDS/PAGE gels were run for E coli lysates containing the plasmids pHCV-29 (Figure 10,), pHCV-23 (Figure 11) ), and pHCV-31 (Figure 12) expressing the recombinant fusion proteins CKS-33c, CKS-BCD, and CKS-33-BCD, respectively. For all 1 three figures, molecular weight standards were run in Lane M, with the arrows on the left indicating the mobilities of the molecular weight markers from top to bottom: 110,000; 84,000; 47,000; 33,000; 24,000; and 16,000 daltons. In Figure 26, Lane 1 contained the E. coti lysate containing pHCV-29 expressing HCV CKS-33c (amino acids 1192 to 1457) prior to induction and lane 2 after 4 hours induction. These results show that the recombinant pHCV-29 fusion protein has an apparent mobility corresponding to a molecular size of 60,000 daltons. This compares acceptably to the predicted molecular mass of 54,911.

In Figure 11 , Lane 1 contained the E.coli lysate containing pJ0200-the CKS vector without the HCV sequence. Lane 2, contained pHCV-20 expressing the HCV CKS-B (amino acids 1676 to 1790). Lane 3, contained the fusion protein pHCV-23 (amino acids 1676-1931). These results show that the recombinant pHCV-23 fusion protein has an apparent mobility corresponding to a molecular size of 55,000 daltons. This compares acceptably to the predicted molecular mass of 55,070 daltons.

In Figure 12 , Lane 1 contained the E,coli lysate containing pJ0200 the CKS vector without the HCV sequences. Lane 2 contained pHCV-31 expressing the CKS-33c-8CD fusion protein (amino acids 1192 to 1457 and 1676 to 1931) prior to induction and lane 3 after 2 hours induction. These results show that the recombinant pHCV-31 ( CKS-33c-BCD) fusion protein has an apparent mobility corresponding to a molecular size of 90,000 daltons. This compares acceptably to the predicted molecular mass of 82,995 daltons.

An immunoblot was also run on one of the SDS/PAGE gels derived from the pHCV-31/X-1 culture. Human serum from an HCV exposed individual reacted strongly with the major pHCV-31 band at 90,000 daltons. Normal human serum did not react with any component of the pHCV-31 (CKS-33-BCD) preparations.

## Claims (Claims for the following Contracting State(s): AT, BE, CH, DE, FR, GB, IT, LI, NL)

1. A method for expressing HCV protein in a microbe, said method **characterized by** the steps of:
(a) providing a DNA vector **characterized by** a plasmid having a lac control region, a region encoding at least a portion of CKS protein in proper reading frame with respect to said control region, and a region encoding said HCV protein, said control region directing expression of said coding regions;
(b) transforming said microbe with said DNA vector; and
(c) expressing a fusion protein of at least a portion of CKS and said HCV protein.

2. The method of Claim 1 **characterized in that** said DNA region encoding said HCV protein is adjacent said DNA region encoding at least a portion of CKS protein.

3. The method of Claim 1 **characterized in that** said DNA region encoding said HCV protein is constructed by bridge mutagenesis of FokI method of gene synthesis.

4. A cloning vector for transformed cells to express heterologous HCV protein, said cloning vector **characterized by** a plasmid having a control region which is a modified lac promoter, a region encoding at least a portion of CKS, and a region encoding for said HCV protein.

5. The cloning vector of Claim 4 **characterized in that** said HCV protein coding region is adjacent said CKS coding region.

6. A fusion protein **characterized in that** at least a portion of the bacterial enzyme CKS is fused to HCV protein consisting of HCV amino acids 1192 to 1457 and 1676 to 1931.

7. A gene sequence for insertion into a plasmid vector, said gene sequence **characterized by**:
(a) a modified lac promoter;
(b) a ribosome binding site;
(c) a first gene subsequence encoding at least a portion of CKS; and
(d) a second gene subsequence encoding a HCV protein sequence.

## Claims (Claims for the following Contracting State(s): DK, GR, SE)

1. A method for expressing HCV protein in a microbe, said method **characterized by** the steps of:
(a) providing a DNA vector having a control region, a region encoding at least a portion of CKS protein, and a region encoding said HCV protein, said control region directing expression of said coding regions;
(b) transforming said microbe with said DNA vector; and
(c) expressing a fusion protein of at least a portion of CKS and said HCV protein.

2. The method of Claim 1 **characterized in that** said DNA vector is provided by:
(a) providing plasmid DNA having a lac control region;
(b) inserting a gene encoding at least a portion of CKS under the control of and in proper reading frame with respect to said control region; and
(c) inserting a DNA region encoding for said protein adjacent said CKS protein, said DNA region encoding for said HCV protein being under the control of said control region.

3. The method of Claim 2 **characterized in that** said DNA region encoding said HCV protein is constructed by bridge mutagenesis of FokI method of gene synthesis.

4. A cloning vector for transformed cells to express heterologous HCV protein, said cloning vector **characterized by** a plasmid having a control region, a region encoding at least a portion of CKS, and a region encoding for said HCV protein.

5. The cloning vector of Claim 4 **characterized in that** said control region is a modified lac promoter.

6. The cloning vector of Claim 4 **characterized in that** said HCV protein coding region is adjacent said CKS coding region.

7. A fusion protein **characterized in that** at least a portion of the bacterial enzyme CKS is fused to HCV protein.

8. A fusion protein **characterized in that** at least a portion of the bacterial enzyme CKS is fused to HCV protein consisting of HCV amino acids 1192 to 1457 and 1676 to 1931.

9. A gene sequence for insertion into a plasmid vector, said gene sequence **characterized by**: (a) a promoter;
(b) a ribosome binding site;
(c) a first gene subsequence encoding at least a portion of CKS; and
(d) a second gene subsequence encoding a HCV protein sequence.

## Claims (Claims for the following Contracting State(s): ES)

1. A method for expressing HCV protein in a microbe, said method **characterized by** the steps of:
(a) providing a DNA vector **characterized by** a plasmid having a lac control region, a region encoding at least a portion of CKS protein in proper reading frame with respect to said control region, and a region encoding said HCV protein, said control region directing expression of said coding regions;
(b) transforming said microbe with said DNA vector; and
(c) expressing a fusion protein of at least a portion of CKS and said HCV protein.

2. The method of Claim 1 **characterized in that** said DNA region encoding said HCV protein is adjacent said DNA region encoding at least a portion of CKS protein.

3. The method of Claim 1 **characterized in that** said DNA region encoding said HCV protein is constructed by bridge mutagenesis of FokI method of gene synthesis.

4. The method of Claim 1 **characterized in that** said HCV protein consists of HCV amino acids 1192 to 1457 and 1676 to 1931.

## Patentansprüche (Patentansprüche für folgende(n) Vertragsstaat(en): DK, GR, SE)

1. Ein Verfahren zur Expression von einem HCV-Protein in einer Mikrobe, wobei das Verfahren durch folgende Schritte **gekennzeichnet** ist:
(a) Bereitstellen eines DNA-Vektors mit einer Kontrollregion, einer Region, die mindestens einen Teil des CKS-Proteins kodiert, und einer Region, die das HCV-Protein kodiert, wobei die Kontrollregion die Expression der kodierenden Regionen steuert;
(b) Transformation der Mikrobe mit dem DNA-Vektor; und
(c) Expression eines Fusionsproteins aus mindestens einem Teil von CKS und dem HCV-Protein.

2. Das Verfahren gemäß Anspruch 1, **dadurch gekennzeichnet, daß** der DNA-Vektor bereitgestellt wird durch:
(a) Bereistellen von Plasmid DNA mit einer lac Kontrollregion;
(b) Insertion eines Gens, das mindestens einen Teil von CKS unter der Kontrolle von und in einem geeigneten Leseraster bezüglich der Kontrollregion kodiert; und
(c) Insertion einer DNA-Region, die für das Protein kodiert, das an das CKS-Protein angrenzend ist, wobei die DNA-Region, die für ' das HCV-Protein kodiert, unter der Kontrolle der Kontrollregion ist.

3. Das Verfahren gemäß Anspruch 2, **dadurch gekennzeichnet, daß** die DNA-Region, die das HCV-Protein kodiert, durch Brückenmutagenese des FokI -Verfahrens der Gensynthese gebildet wird.

4. Ein Klonierungsvektor für transformierte Zellen, um ein heterologes HCV-Protein zu exprimieren, wobei der Klonierungsvektor charakterisiert ist durch ein Plasmid mit einer Kontrollregion, einer Region, die mindestens einen Teil von CKS kodiert, und einer Region; die für das HCV-Protein kodiert.

5. Der Klonierungsvektor gemäß Anspruch 4, **dadurch gekennzeichnet, daß** die Kontrollregion ein modifizierter lac Promoter ist.

6. Der Klonierungsvektor gemäß Anspruch 4, **dadurch gekennzeichnet, daß** die HCV-Protein kodierende Region an die CKS kodierende Region angrenzend ist.

7. Ein Fusionsprotein, **dadurch gekennzeichnet, daß** mindestens ein Teil des bakteriellen Enzyms CKS an ein HCV-Protein ankondensiert ist.

8. Ein Fusionsprotein, **dadurch gekennzeichnet, daß** mindestens ein Teil des bakteriellen Enzyms CKS an ein HCV-Protein, bestehend aus HCV-Aminosäure 1192 bis 1457 und 1676 bis 1931, ankondensiert ist.

9. Eine Gensequenz zur Insertion in einen Plasmidvektor, wobei die Gensequenz **gekennzeichnet ist durch**:
(a) einen Promoter;
(b) eine Ribosombindungsstelle;
(c) eine erste Gensubsequenz, die mindestens einen Teil von CKS kodiert; und
(d) eine zweite Gensubsequenz, die eine HCV-Proteinsequenz kodiert.

## Patentansprüche (Patentansprüche für folgende(n) Vertragsstaat(en): ES)

1. Ein Verfahren zur Expression von einem HCV-Protein in einer Mikrobe, wobei das Verfahren durch folgende Schritte **gekennzeichnet** ist:
(a) Bereitstellen eines DNA-Vektors, charakterisiert durch ein Plasmid mit einer lac Kontrollregion, einer Region, die mindestens einen Teil des CKS-Proteins kodiert in einem geeigneten Leseraster bezüglich der Kontrollregion, und einer Region, die das HCV-Protein kodiert, wobei die Kontrollregion die Expression der kodierenden Regionen steuert;
(b) Transformation der Mikrobe mit dem DNA-Vektor; und
(c) Expression eines Fusionsproteins aus mindestens einem Teil von CKS und dem HCV-Protein.

2. Das Verfahren gemäß Anspruch 1, **dadurch gekennzeichnet, daß** die DNA-Region, die das HCV-Protein kodiert, an die DNA-Region angrenzend ist, die mindestens einen Teil des CKS-Proteins kodiert.

3. Das Verfahren gemäß Anspruch 1, **dadurch gekennzeichnet, daß** die DNA-Region, die das HCV-Protein kodiert, durch Brückenmutagenase des FokI -Verfahrens der Gensynthese gebildet wird.

4. Das Verfahren gemäß Anspruch 1, **dadurch gekennzeichnet, daß** das HCV-Protein aus HCV-Aminosäuren 1192 bis 1457 und 1676 bis 1931 besteht.

## Patentansprüche (Patentansprüche für folgende(n) Vertragsstaat(en): AT, BE, CH, DE, FR, GB, IT, LI, NL)

1. Ein Verfahren zur Expression von einem HCV-Protein in einer Mikrobe, wobei das Verfahren durch folgende Schritte **gekennzeichnet** ist:
(a) Bereitstellen eines DNA-Vektors, charakterisiert durch ein Plasmid mit einer lac Kontrollregion, einer Region, die mindestens einen Teil des CKS-Proteins kodiert in einem geeigneten Leseraster bezüglich der Kontrollregion, und einer Region, die das HCV-Protein kodiert, wobei die Kontrollregion die Expression der kodierenden Regionen steuert;
(b) Transformation der Mikrobe mit dem DNA-Vektor; und
(c) Expression eines Fusionsproteins aus mindestens einem Teil von CKS und dem HCV-Protein.

2. Das Verfahren gemäß Anspruch 1, **dadurch gekennzeichnet, daß** die DNA-Region, die das HCV-Protein kodiert, an die DNA-Region angrenzend ist, die mindestens einen Teil des CKS-Proteins kodiert.

3. Das Verfahren gemäß Anspruch 1, **dadurch gekennzeichnet, daß** die DNA-Region, die das HCV-Protein kodiert, durch Brückenmutagenese des FokI -Verfahrens der Gensynthese gebildet wird.

4. Ein Klonierungsvektor für transformierte Zellen, um ein heterologes HCV-Protein zu exprimieren, wobei der Klonierungsvektor charakterisiert ist durch ein Plasmid mit einer Kontrollregion, die ein modifizierter lac Promoter ist, einer Region, die mindestens einen Teil von CKS kodiert, und einer Region, die für das HCV-Protein kodiert.

5. Der Klonierungsvektor gemäß Anspruch 4, **dadurch gekennzeichnet, daß** die HCV-Protein kodierende Region an die CKS kodierende Region angrenzend ist.

6. Ein Fusionsprotein, **dadurch gekennzeichnet, daß** mindestens ein Teil des bakteriellen Enzyms CKS an ein HCV-Protein, bestehend aus HCV-Aminosäuren 1192 bis 1457 und 1676 bis 1931, ankondensiert ist.

7. Eine Gensequenz zur Insertion in einen Plasmidvektor, wobei die Gensequenz **gekennzeichnet ist durch**:
(a) einen modifizierten lac Promoter;
(b) eine Ribosombindungsstelle;
(c) eine erste Gensubsequenz, die mindestens einen Teil von CKS kodiert; und
(d) eine zweite Gensubsequenz, die eine HCV-Proteinsequenz kodiert.

## Revendications (Revendications pour l'(les) Etat(s) contractant(s) suivant(s): DK, GR, SE)

1. Procédé pour exprimer une protéine de HCV dans un microbe, ledit procédé étant **caractérisé par** les étapes de :
(a) obtention d'un vecteur d'ADN ayant une région de contrôle, une région codant au moins une partie d'une protéine CKS et une région codant ladite protéine de HCV, ladite région de contrôle dirigeant l'expression desdites régions codantes ;
(b) transformation dudit microbe avec ledit vecteur d'ADN ; et
(c) expression d'une protéine de fusion d'au moins une partie de CKS et de ladite protéine de HCV.

2. Procédé selon la revendication 1, **caractérisé en ce que** ledit vecteur d'ADN est obtenu par :
(a) obtention d'un ADN plasmidique ayant une région de contrôle lac ;
(b) insertion d'un gène codant au moins une partie de CKS sous le contrôle de et dans un cadre de lecture approprié par rapport à ladite région de contrôle ; et
(c) insertion d'une région d'ADN codant ladite protéine en position adjacente à ladite protéine CKS, ladite région d'ADN codant ladite protéine de HCV étant sous le contrôle de ladite région de contrôle.

3. Procédé selon la revendication 2, **caractérisé en ce que** ladite région d'ADN codant ladite protéine de HCV est construite par un procédé de synthèse génique par mutagenèse par pontage de FokI.

4. Vecteur de clonage pour des cellules transformées pour exprimer une protéine de HCV hétérologue, ledit vecteur de clonage étant **caractérisé par** un plasmide ayant une région de contrôle, une région codant au moins une partie de CKS et une région codant ladite protéine de HCV.

5. Vecteur de clonage selon la revendication 4, **caractérisé en ce que** ladite région de contrôle est un promoteur lac modifié.

6. Vecteur de clonage selon la revendication 4, **caractérisé en ce que** ladite région codant la protéine de HCV est adjacente à ladite région codant CKS.

7. Protéine de fusion **caractérisée en ce qu'**au moins une partie de l'enzyme bactérienne CKS est fusionnée à une protéine de HCV.

8. Protéine de fusion **caractérisée en ce qu'**au moins une partie de l'enzyme bactérienne CKS est fusionnée à une protéine de HCV consistant en les acides aminés de HCV 1192 à 1457 et 1676 à 1931.

9. Séquence génique destinée à être insérée dans un vecteur plasmidique, ladite séquence génique étant **caractérisée par** :
(a) un promoteur ;
(b) un site de fixation ribosomique ;
(c) une première sous-séquence génique codant au moins une partie de CKS ; et
(d) une seconde sous-séquence génique codant une séquence de protéine de HCV.

## Revendications (Revendications pour l'(les) Etat(s) contractant(s) suivant(s): ES)

1. Procédé pour exprimer une protéine de HCV dans un microbe, ledit procédé étant **caractérisé par** les étapes de :
(a) obtention d'un vecteur d'ADN **caractérisé par** un plasmide ayant une région de contrôle lac, une région codant au moins une partie d'une protéine CKS dans un cadre de lecture approprié par rapport à ladite région de contrôle, et une région codant ladite protéine de HCV, ladite région de contrôle dirigeant l'expression desdites régions codantes ;
(b) transformation dudit microbe avec ledit vecteur d'ADN ; et
(c) expression d'une protéine de fusion d'au moins une partie de CKS et de ladite protéine de HCV.

2. Procédé selon la revendication 1, **caractérisé en ce que** ladite région d'ADN codant ladite protéine de HCV est adjacente à ladite région d'ADN codant au moins une partie de la protéine CKS.

3. Procédé selon la revendication 1, **caractérisé en ce que** ladite région d'ADN codant ladite protéine de HCV est construite par un procédé de synthèse génique par mutagenèse par pontage de FokI.

4. Procédé selon la revendication 1, **caractérisé en ce que** ladite protéine de HCV consiste en les acides aminés de HCV 1192 à 1457 et 1676 à 1931.

## Revendications (Revendications pour l'(les) Etat(s) contractant(s) suivant(s): AT, BE, CH, DE, FR, GB, IT, LI, NL)

1. Procédé pour exprimer une protéine de HCV dans un microbe, ledit procédé étant **caractérisé par** les étapes de :
(a) obtention d'un vecteur d'ADN **caractérisé par** un plasmide ayant une région de contrôle lac, une région codant au moins une partie d'une protéine CKS dans un cadre de lecture approprié par rapport à ladite région de contrôle, et une région codant ladite protéine de HCV, ladite région de contrôle dirigeant l'expression desdites régions codantes ;
(b) transformation dudit microbe avec ledit vecteur d'ADN ; et
(c) expression d'une protéine de fusion d'au moins une partie de CKS et de ladite protéine de HCV.

2. Procédé selon la revendication 1, **caractérisé en ce que** ladite région d'ADN codant ladite protéine de HCV est adjacente à ladite région d'ADN codant au moins une partie de la protéine CKS.

3. Procédé selon la revendication 1, **caractérisé en ce que** ladite région d'ADN codant ladite protéine de HCV est construite par un procédé de synthèse génique par mutagenèse par pontage de FokI.

4. Vecteur de clonage pour des cellules transformées pour exprimer une protéine de HCV hétérologue, ledit vecteur de clonage étant **caractérisé par** un plasmide ayant une région de contrôle qui est un promoteur lac modifié, une région codant au moins une partie de CKS et une région codant ladite protéine de HCV.

5. Vecteur de clonage selon la revendication 4, **caractérisé en ce que** ladite région codant la protéine de HCV est adjacente à ladite région codant CKS.

6. Protéine de fusion **caractérisée en ce qu'**au moins une partie de l'enzyme bactérienne CKS est fusionnée à une protéine de HCV consistant en les acides aminés de HCV 1192 à 1457 et 1676 à 1931.

7. Séquence génique destinée à être insérée dans un vecteur plasmidique, ladite séquence génique étant **caractérisée par** :
(a) un promoteur lac modifié ;
(b) un site de fixation ribosomique ;
(c) une première sous-séquence génique codant au moins une partie de CKS ; et
(d) une seconde sous-séquence génique codant une séquence de protéine de HCV.
